# EUROPEAN PATENT SPECIFICATION

(11) **EP 3 509 635 B1**
(45) Date of publication and mention of the grant of the patent: **11.11.2026**
(21) Application number: 17784049.3
(22) Date of filing: 05.09.2017
(51) Int. Cl.: A61K 39/395, A61K 38/38, A61K 9/51, A61K 31/337, A61P 35/00, C07K 16/22, C07K 16/28, C07K 16/32

(54) **METHODS OF TREATING TRIPLE-NEGATIVE BREAST CANCER USING COMPOSITIONS OF ANTIBODIES AND CARRIER PROTEINS**
VERFAHREN ZUR BEHANDLUNG VON DREIFACH NEGATIVEM BRUSTKREBS UNTER VERWENDUNG VON ZUSAMMENSETZUNGEN AUS ANTIKÖRPERN UND TRÄGERPROTEINEN
MÉTHODES DE TRAITEMENT D'UN CANCER DU SEIN TRIPLE-NÉGATIF À L'AIDE DE COMPOSITIONS D'ANTICORPS ET DE PROTÉINES PORTEUSES

(30) Priority: 06.09.2016 US 201662383943 P
(43) Date of publication of application: 17.07.2019
(73) Proprietor: Vavotar Life Sciences LLC, Cumming, GA 30041 (US); Mayo Foundation for Medical Education and Research, Rochester, Minnesota 55905 (US)
(72) Inventor: SWISS, Gerald F., Cumming, Georgia 30041 (US); CROWNE, Jesse, Cumming, Georgia 30041 (US); MARKOVIC, Svetomir N., Rochester, Minnesota 55905 (US); NEVALA, Wendy K., Rochester, Minnesota 55905 (US)
(74) Representative: Fish & Richardson P.C.
(86) International application number: PCT/US2017/050134
(87) International publication number: WO 2018/048815

(56) References cited:
- WO-A1-2016/057554
- US-A1- 2011 104 143
- MATSUDA NAOKO ET AL: "Phase II study of panitumumab, nab-paclitaxel, and carboplatin followed by FEC neoadjuvant chemotherapy for patients with primary HER-2 negative inflammatory breast cancer.", JOURNAL OF CLINICAL ONCOLOGY, vol. 33, no. 15_suppl, 20 May 2015 (2015-05-20), pages 1065, XP093028818, Retrieved from the Internet <URL:https://ascopubs.org/doi/abs/10.1200/jco.2015.33.15_suppl.1065>
- MATSUDA NAOKO ET AL: "Phase II study of panitumumab, nab-paclitaxel, and carboplatin followed by FEC neoadjuvant chemotherapy for patients with primary HER2-negative inflammatory breast cancer.", JOURNAL OF CLINICAL ONCOLOGY, vol. 34, no. 15_suppl, 20 May 2016 (2016-05-20), pages 1087, XP93028971, Retrieved from the Internet <URL:https://ascopubs.org/doi/10.1200/JCO.2016.34.15_suppl.1087>
- BUDHI S YADAV: "Biomarkers in triple negative breast cancer: A review", WORLD JOURNAL OF CLINICAL ONCOLOGY, vol. 6, no. 6, 1 January 2015 (2015-01-01), US, pages 252, XP055577439, ISSN: 2218-4333, DOI: 10.5306/wjco.v6.i6.252
- KALIMUTHO MURUGAN ET AL: "Targeted Therapies for Triple-Negative Breast Cancer: Combating a Stubborn Disease", TRENDS IN PHARMACOLOGICAL SCIENCES, vol. 36, no. 12, December 2015 (2015-12-01), pages 822 - 846, XP029339482, ISSN: 0165-6147, DOI: 10.1016/J.TIPS.2015.08.009
- LISA D VOLK-DRAPER ET AL: "Novel Model for Basaloid Triple-negative Breast Cancer: Behavior In Vivo and Response to Therapy", NEOPLASIA, vol. 14, no. 10, 1 October 2012 (2012-10-01), US, pages 926 - IN13, XP055422437, ISSN: 1476-5586, DOI: 10.1593/neo.12956
- GIORGIO MUSTACCHI ET AL: "The role of taxanes in triple-negative breast cancer: literature review", DRUG DESIGN, DEVELOPMENT AND THERAPY, vol. 9, 5 August 2015 (2015-08-05), pages 4303 - 4318, XP055422380, DOI: 10.2147/DDDT.S86105
- ERIKA HAMILTON ET AL: "Nab-Paclitaxel/Bevacizumab/Carboplatin Chemotherapy in First-Line Triple Negative Metastatic Breast Cancer", CLINICAL BREAST CANCER, vol. 13, no. 6, 1 December 2013 (2013-12-01), US, pages 416 - 420, XP055422364, ISSN: 1526-8209, DOI: 10.1016/j.clbc.2013.08.003
- NAHLEH Z A ET AL: "SWOG S0800 (NCI CDR0000636131): addition of bevacizumab to neoadjuvant nab-paclitaxel with dose-dense doxorubicin and cyclophosphamide improves pathologic complete response (pCR) rates in inflammatory or locally advanced breast cancer", BREAST CANCER RESEARCH AND TREATMENT, SPRINGER , NY, US, vol. 158, no. 3, 8 July 2016 (2016-07-08), pages 485 - 495, XP036016293, ISSN: 0167-6806, [retrieved on 20160708], DOI: 10.1007/S10549-016-3889-6
- SYLVIA ADAMS ET AL: "Phase Ib trial of atezolizumab in combination with nab-paclitaxel in patients with metastatic triple-negative breast cancer (mTNBC)", JOURNAL OF CLINICAL ONCOLOGY, vol. 34, no. 15_suppl, 1 May 2016 (2016-05-01), pages 1009 - 1009, XP055422710, DOI: 10.1200/JCO.2016.34.15_suppl.1009
- ALESSANDRA FABI ET AL: "Prospective study on nanoparticle albumin-bound paclitaxel in advanced breast cancer: clinical results and biological observations in taxane-pretreated patients", DRUG DESIGN, DEVELOPMENT AND THERAPY, vol. 9, 1 November 2015 (2015-11-01), United Kingdom, pages 6177 - 6183, XP055423041, ISSN: 1177-8881, DOI: 10.2147/DDDT.S89575

## Description

### FIELD OF THE INVENTION

This disclosure relates to novel methods and kits for treating triple-negative breast cancer using nanoparticle complexes.

### BACKGROUND

Breast cancer is the most common malignancy and second leading cause of cancer death among women in the United States, with an estimated 192,370 new cases diagnosed and 40,170 deaths reported in 2009. Among women aged 20 to 59 years, breast cancer remains the leading cause of cancer death despite a steady decrease in breast cancer mortality since 1990. Triple-negative breast cancer (TNBC) is an aggressive clinical phenotype characterized by lack of expression (or minimal expression) of estrogen receptor (ER) and progesterone receptor (PR), as well as an absence of human epidermal growth factor receptor-2 (Her2/neu) overexpression. For example, a 2007 study of more than 50,000 women with breast cancer found that 23% of women with triple-negative breast cancer did not survive past 5 years, versus 7% of women with other types of breast cancer. Unlike patients with ER/PR-positive or HER2-overexpressing cancer, systemic treatment options for patients with TNBC are limited to cytotoxic chemotherapy due to the lack of a molecular target. Despite its chemosensitivity, TNBC is still associated with a poor prognosis. Pal, S. et al. (2011) Breast Cancer Res Treat. 125: 627-636.

Thus, there remains a need in the art to improve the efficacy of cancer therapeutics, especially for the treatment of triple-negative breast cancer.

Mustacchi and De Laurentiis (*Drug design, development and therapy* (2015)) describes the role of taxanes in triple-negative breast cancer.

WO 2016/057554 describes compositions of antibodies and carrier proteins and methods of making and using the same, in particular, as a cancer therapeutic, and lyophilized compositions of antibodies and carrier proteins and methods of making and using the same, in particular, as a cancer therapeutic.

Adams, et al. (Journal of Clinical Oncology (2016)) describes a phase Ib trial of atezolizumab in combination with nab-paclitaxel in patients with metastatic triple-negative breast cancer (mTNBC).

### SUMMARY

Disclosed herein are compositions for use in methods for treating a patient afflicted with breast cancer characterized as having a triple negative phenotype, e.g., a breast cancer characterized as estrogen receptor negative, progesterone receptor negative and Her2/neu negative, and further characterized as expressing a target antigen, e.g., VEGF. A composition for use in a method of this invention comprises a composition for use in a method of treating a breast cancer cell characterized as a triple negative phenotype, which method comprises administering to a breast cancer cell characterized as a triple negative phenotype a composition comprising a therapeutic amount of nanoparticle complexes, the complexes comprising: a) albumin; b) an effective amount of an antibody that binds to the antigen expressed by said cells of said breast cancer so as to provide directional guidance of the nanoparticle complexes to said cells; and c) an effective amount of paclitaxel, wherein the antigen expressed by said cells is cancer testis (CT) antigen, folate receptor alpha (FR-alpha), programmed-death-ligand 1 (PD-L1), programmed cell death protein 1 (PD-1), epidermal growth factor receptor (EGFR), C-kit, and/or basal cytokeratin, wherein the antibody is selected from an anti-cancer testis (CT) antigen antibody, an anti-folate receptor alpha (FR-alpha) antibody, an anti-programmed death-ligand 1 (PD-L1) antibody, an anti-programmed cell death protein 1 (PD-1) antibody, an anti-epidermal growth factor receptor (EGFR) antibody, an anti-C-kit antibody, and an anti-basal cytokeratin antibody, and wherein said antibodies are arranged on the outside surface of the nanoparticle complexes.

The nanoparticle composition useful in the methods disclosed herein may be lyophilized. Upon reconstitution of such lyophilized composition with an aqueous solution, antigen-binding portions of the binding agents are arranged on the outside surface of the nanoparticle complexes and the nanoparticle complexes are capable of binding to the target antigen, e.g., VEGF, *in vivo.*

The nanoparticle complexes described herein may comprise paclitaxel in combination with one or more additional chemotherapeutic agents, e.g., a chemotherapeutic agent selected from the group consisting of abiraterone, bendamustine, bortezomib, carboplatin, cabazitaxel, cisplatin, chlorambucil, dasatinib, docetaxel, doxorubicin, epirubicin, erlotinib, etoposide, everolimus, gefitinib, idarubicin, imatinib, hydroxyurea, imatinib, lapatinib, leuprorelin, melphalan, methotrexate, mitoxantrone, nedaplatin, nilotinib, oxaliplatin, pazopanib, pemetrexed, picoplatin, romidepsin, satraplatin, sorafenib, vemurafenib, sunitinib, teniposide, triplatin, vinblastine, vinorelbine, vincristine, and cyclophosphamide.

The carrier protein useful in the methods described herein is albumin. The albumin may be, e.g., human serum albumin or a recombinant human serum albumin. Recombinant human serum albumin is a highly purified and animal-, virus-, and bacteria-free product that was developed as an alternative to plasma-derived human serum albumin.

In one aspect, the nanoparticle complexes useful in the methods described herein contain binding agents, e.g., antibodies, arranged into a substantially single layer on all or part of the surface of the nanoparticle. In an aspect of this invention, each nanoparticle complex comprises between 100 and 1,000 antibodies, e.g., between 400 and 800 antibodies. In some embodiments the average nanoparticle complex size in the composition is from greater than 800 nm to about 3.5 µm. In other embodiments, the average size of the nanoparticle complexes is about 100 nm to less than about 1 micron. In another aspect the average size of the nanoparticle complexes is between about 120 nm to about 800 nm, and between 120 nm and 200 nm. In some embodiments the nanoparticle complex has an average size of about 160 nm. The nanoparticle complex sizes as set forth herein are as measured with light scatter technology using a Mastersizer 2000E (Malvern Instruments Ltd., Worcestershire, England).

In an embodiment, the carrier protein is albumin and the therapeutic agent is paclitaxel, said nanoparticle complexes having an average size of approximately 80 nm to 400 nm, of about 120 nm to about 200 nm, preferably approximately 160 nm.

In an embodiment of the compositions for use in methods of this invention, the patient has not previously been treated with a therapeutic dose of anti-VEGF antibody prior to administration of the nanoparticle composition. In another embodiment, the patient is pretreated with an anti-VEGF antibody prior to administration of the nanoparticle composition. In one embodiment, the anti-VEGF antibody is administered in a subtherapeutic dose prior to administration of the nanoparticle composition.

In some cases the patient is recalcitrant to conventional hormonal therapies, and/or trastuzumab therapies.

In an embodiment of the methods of this invention, the composition is administered in a dose that provides 75-175 mg/m² of the paclitaxel to the patient.

As an example not forming part of the claimed invention, provided herein are compositions for use in a method for treating a patient afflicted with breast cancer characterized as estrogen receptor negative, progesterone receptor negative and Her2/neu negative and further characterized as expressing VEGF, said method comprises treating said patient with a composition comprising a therapeutic amount of nanoparticle complexes, said complexes comprising: a) human serum albumin; b) bevacizumab or a biosimilar version thereof; and c) paclitaxel.

In an embodiment of the methods of this invention, the ratio of human serum albumin to paclitaxel in the nanoparticles is about 1:1 to about 13:1, about 5:1 to about 9:1, or about 9:1.

In an embodiment of the methods of this invention, the ratio of albumin/paclitaxel to antibody is about 10:1 to about 1:10, or about 5:1 to about 1:5.

### DETAILED DESCRIPTION

After reading this description it will become apparent to one skilled in the art how to implement the invention in various alternative embodiments and alternative applications.

However, all the various embodiments of the present invention will not be described herein. It will be understood that the embodiments presented here are presented by way of an example only, and not limitation. As such, this detailed description of various alternative embodiments should not be construed to limit the scope or breadth of the present invention. The extent of the scope of the present invention is defined by the claims.

Before the present invention is disclosed and described, it is to be understood that the aspects described below are not limited to specific compositions, methods of preparing such compositions, or uses thereof as such may, of course, vary. It is also to be understood that the terminology used herein is for the purpose of describing particular aspects only and is not intended to be limiting.

The detailed description of the invention is divided into various sections only for the reader's convenience and disclosure found in any section may be combined with that in another section. Titles or subtitles may be used in the specification for the convenience of a reader, which are not intended to influence the scope of the present invention.

Throughout this specification "VEGF" and "anti-VEGF antibody" are used as examples and it is appreciated that other target antigens, e.g., other cytokines and other chemokines, that are expressed by the triple negative breast cancer cells and other antibodies to such antigens may also be used in the complexes and methods described herein.

### Overview

This invention is directed to compositions for use in methods of treating a mammal with breast cancer, characterized as having a triple negative phenotype, e.g., triple-negative breast cancer characterized as estrogen receptor-negative, progesterone receptor-negative and Her2/neu- negative, and is further characterized as expressing a target antigen.

A composition for use in a method of this invention comprises a composition for use in a method of treating a breast cancer cell characterized as a triple negative phenotype, which method comprises administering to a breast cancer cell characterized as a triple negative phenotype a composition comprising a therapeutic amount of nanoparticle complexes, the complexes comprising: a)albumin; b) an effective amount of an antibody that binds to the antigen expressed by said cells of said breast cancer so as to provide directional guidance of the nanoparticle complexes to said cells; and c) an effective amount of paclitaxel, wherein the antigen expressed by said cells is cancer testis (CT) antigen, folate receptor alpha (FR-alpha), programmed-death-ligand 1 (PD-L1), programmed cell death protein 1 (PD-1), epidermal growth factor receptor (EGFR), C-kit, and/or basal cytokeratin, wherein the antibody is selected from an anti-cancer testis (CT) antigen antibody, an anti-folate receptor alpha (FR-alpha) antibody, an anti-programmed death-ligand 1 (PD-L1) antibody, an anti-programmed cell death protein 1 (PD-1) antibody, an anti-epidermal growth factor receptor (EGFR) antibody, an anti-C-kit antibody, and an anti-basal cytokeratin antibody, and wherein said antibodies are arranged on the outside surface of the nanoparticle complexes.

The materials provided herein can be used in a method to increase the progression- free survival rate in patients with breast cancer, characterized as having a triple negative phenotype. Increasing progression-free survival can improve a patient's quality of life and/or allow breast cancer patients to live longer.

The materials provided herein can be used in a method to increase the overall survival rate in triple breast cancer patients.

As an example not forming part of the claimed invention, methods for treating a patient suffering from triple-negative breast cancer that expresses a target antigen, e.g., VEGF, by treating the patient with a composition comprising a therapeutically effective amount of nanoparticle complexes comprising a carrier protein, e.g., albumin, paclitaxel, and a binding agent that specifically binds to the target antigen (e.g., an anti-VEGF antibody).

The example methods also relate to treating a patient suffering from triple-negative breast cancer that comprise cancer cells that expresses a target antigen, e.g., VEGF, with a subtherapeutic amount of binding agent that specifically binds to the target antigen, (e.g., anti-VEGF antibody) and a composition comprising a therapeutically effective amount of nanoparticle complexes comprising a carrier protien (e.g., albumin), paclitaxel and a binding agent that specifically binds to the target antigen (e.g., anti-VEGF antibody). Unless otherwise defined, all technical and scientific terms used herein have the same meaning as commonly understood by one of ordinary skill in the art to which this invention pertains.

In case of conflict between the present specification and a reference mentioned herein, the present specification, including definitions, will control. In addition, the materials, methods, and examples are illustrative only and not intended to be limiting.

Throughout the specification "VEGF" and "anti-VEGF antibody" are used in an exemplary fashion and it will be understood that other target antigens, e.g., other cytokines and chemokines, that are expressed by the triple negative breast cancer cells and other antibodies to those antigens may also be used in the complexes and methods described herein.

### Definitions

In this specification and in the claims that follow, reference will be made to a number of terms that shall be defined to have the following meanings:
The terminology used herein is for the purpose of describing particular embodiments only and is not intended to be limiting of the invention. As used herein, the singular forms "a", "an" and "the" are intended to include the plural forms as well, unless the context clearly indicates otherwise.

"Optional" or "optionally" means that the subsequently described event or circumstance can or cannot occur, and that the description includes instances where the event or circumstance occurs and instances where it does not.

The term "about" when used before a numerical designation, e.g., temperature, time, amount, concentration, and such other, including a range, indicates approximations which may vary by (+) or ( - ) 10%, 5%, 1%, or any sub-range or sub-value there between. Preferably, the term "about" when used with regard to a dose amount means that the dose may vary by +/- 10%.

"Comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the claimed invention. "Consisting of" shall mean excluding more than trace elements of other ingredients and substantial method steps. Embodiments defined by each of these transition terms are within the scope of this invention.

Heretofore, the use of bevacizumab in the treatment of breast cancer was discontinued due to FDA decisions based on the lack of sufficient efficacy of this antibody in this treatment protocol. ABRAXANE^{®}, while approved for metastatic breast cancer treatment, provides an adequate therapy. However, the compositions for use in a method to treat breast cancer described herein are contemplated to provide a significantly improved therapy, especially for the difficult-to-treat triple negative breast cancer where mortality rates are unacceptably high. Without being limited to any theory, it is believed the directional guidance provided by the complexation of the antibody in the nanoparticle complexes provides unexpected results.

In view of the foregoing it is contemplated that subtherapeutic amounts of either the chemotherapeutic agent or the antibody may be successfully used in the treatments described herein. As used herein, the term "sub-therapeutic" is used to describe an amount of chemotherapeutic agent or antibody that is below the amount of agent or antibody conventionally used to treat a cancer. For example, a sub-therapeutic amount is an amount less than that defined by the manufacturer as being required for therapy.

The term "nanoparticle" or "nanoparticle complex" as used herein refers to particles having at least one dimension which is less than 5 microns. In preferred embodiments, such as for intravenous administration, the particle is less than 1 micron. For direct administration, e.g., into a tumor, the particle can be larger. Even larger particles are expressly contemplated by the invention.

In a population of particles, the size of individual particles is distributed about a mean. Particle sizes for the population can therefore be represented by an average, and also by percentiles. D50 is the particle size below which 50% of the particles fall. 10% of particles are smaller than the D10 value and 90% of particles are smaller than D90. Where unclear, the "average" size is equivalent to D50. So, for example, AB160 refers to nanoparticles having an average size of 160 nanometers.

The term "nanoparticle" may also encompass discrete multimers of smaller unit nanoparticles. For example, a 320 nm particle may comprise a dimer of a unit 160 nm nanoparticle. For 160 nm nanoparticles, multimers would therefore be approximately 320 nm, 480 nm, 640 nm, 800 nm, 960 nm, 1120 nm, and so on as determined by a Mastersizer 2000 (available from Malvern Instruments Ltd, Worcestershire, UK) as described in WO 2016/057554 A1.

The term "binding agent" as used herein refers to any compound having an antigen-binding portion specific to an antigen expressed by a cancer cell of interest that binds to said antigen with high specificity, for example having a dissociation constant of 10⁻⁷M, 10⁻⁸M, or 10⁻⁹M or lower. Preferably the dissociation constant is about 10⁻⁷ M to about 10⁻¹⁴M. Examples of binding agents that can be effectively employed in the disclosed methods include, but are not limited to, antibodies, such as monoclonal antibodies, e.g., humanized monoclonal antibodies, chimeric antibodies, or polyclonal antibodies, or antigen-binding fragments thereof, as well as aptamers, fusion proteins, and aptamers having or fused to an albumin-binding motif that non-covalently associates with albumin, etc. In an embodiment the binding agent is an exogenous antibody. An exogenous antibody is an antibody not naturally produced in a mammal, e.g., in a human, by the mammalian immune system.

The term "biosimilar" as used herein refers to a biopharmaceutical which is deemed to be comparable in quality, safety, and efficacy to a reference product marketed by an innovator company (see, Section 351(i) of the Public Health Service Act (42 U.S.C. 262(i)).

The term "carrier protein" as used herein refers to proteins that function to transport binding agents and/or therapeutic agents. The binding agents of the present disclosure can reversibly bind to the carrier proteins. Example carrier proteins are discussed in more detail below. Preferably, the carrier protein comprises an antibody-binding motif that non-covalently associates with an antibody.

The term "core" as used herein refers to central or inner portion of the nanoparticle which may be comprised of a carrier protein, a carrier protein and a therapeutic agent, or other agents or combination of agents. In some embodiments, a portion of the binding agent, e.g., an antibody, may be incorporated into the core. In some embodiments, a portion of the binding agent may be associated with (non-covalently bound to) the core.

As used herein, the term "enhancing the therapeutic outcome" and the like relative to a cancer patient refers to a slowing or diminution of the growth of cancer cells or a solid tumor, or a reduction in the total number of cancer cells or total tumor burden.

The term "therapeutic agent" as used herein means an agent which is therapeutically useful, e.g., an agent for the treatment, remission or attenuation of a disease state, physiological condition, symptoms, or etiological factors, or for the evaluation or diagnosis thereof. A therapeutic agent may be a chemotherapeutic agent, for example, mitotic inhibitors, topoisomerase inhibitors, steroids, anti-tumor antibiotics, antimetabolites, alkylating agents, enzymes, proteasome inhibitors, or any combination thereof.

The term "antibody" or "antibodies" as used herein refers to immunoglobulin molecules and immunologically active portions of immunoglobulin molecules (i.e., molecules that contain an antigen binding site that immuno-specifically bind an antigen). The term also refers to antibodies comprised of two immunoglobulin heavy chains and two immunoglobulin light chains as well as a variety of forms including full length antibodies and portions thereof that bind to a target antigen; including, for example, an immunoglobulin molecule, a monoclonal antibody, a chimeric antibody, a CDR-grafted antibody, a humanized antibody, an Fab, an Fab', an F(ab')2, an Fv, a disulfide linked Fv, an scFv, a single domain antibody (dAb), a diabody, a multi-specific antibody, a dual specific antibody, an anti-idiotypic antibody, a bispecific antibody, a functionally active target antigen-binding fragment thereof, bifunctional hybrid antibodies (e.g., Lanzavecchia et al., Eur. J. Immunol. 17, 105 (1987)) and single chains (e.g., Huston et al., Proc. Natl. Acad. Sci. U.S.A., 85, 5879-5883 (1988) and Bird et al., Science 242, 423-426 (1988)). (See, generally, Hood et al., Immunology, Benjamin, N.Y., 2nd ed. (1984); Harlow and Lane, Antibodies. A Laboratory Manual, Cold Spring Harbor Laboratory (1988); Hunkapiller and Hood, Nature, 323, 15-16 (1986)). The antibody may be of any type (e.g., IgG, IgA, IgM, IgE or IgD). Preferably, the antibody is IgG. An antibody may be nonhuman (e.g., from mouse, goat, or any other animal), fully human, humanized, or chimeric. In an embodiment the antibody is an exogenous antibody. An exogenous antibody is an antibody not naturally produced in a mammal, e.g., in a human, by the mammalian immune system.

The term "dissociation constant," also referred to as "K_{d}," refers to a quantity expressing the extent to which a particular substance separates into individual components (e.g., the protein carrier, antibody, and optional therapeutic agent).

The terms "lyophilized," "lyophilization" and the like as used herein refer to a process by which the material (e.g., nanoparticle complexes) to be dried is first frozen and then the ice or frozen solvent is removed by sublimation in a vacuum environment. An excipient is optionally included in pre-lyophilized formulations to enhance stability of the lyophilized product upon storage. In some embodiments, the nanoparticle complexes can be formed from individual lyophilized components (carrier protein, antibody, and optional therapeutic agent) prior to use as a therapeutic. In other embodiments, the carrier protein, antibody, and optional therapeutic agent are first combined into nanoparticle complexes and then lyophilized. The lyophilized sample may further contain additional excipients.

The term "buffer" encompasses those agents which maintain the solution pH in an acceptable range prior to lyophilization and may include succinate (sodium or potassium), histidine, phosphate (sodium or potassium), Tris (tris(hydroxymethyl)aminomethane), diethanolamine, citrate (sodium) and the like. The buffer of this invention has a pH in the range from about 5.5 to about 6.5; and preferably has a pH of about 6.0. Examples of buffers that will control the pH in this range include succinate (such as sodium succinate), gluconate, histidine, citrate and other organic acid buffers.

The term "pharmaceutical formulation" refers to preparations which are in such form as to permit the active ingredients to be effective, and which contains no additional components that are toxic to the subjects to which the formulation would be administered.

"Pharmaceutically acceptable" excipients (vehicles, additives) are those which can reasonably be administered to a subject mammal to provide an effective dose of the active ingredient employed.

"Reconstitution time" is the time that is required to rehydrate a lyophilized formulation into a solution.

A "stable" formulation is one in which the protein therein essentially retains its physical stability and/or chemical stability and/or biological activity upon storage.

The term "target molecule" or "target antigen" as used herein refers to any molecule expressed by a cancer cell that is recognized and bound by an antibody, an aptamer, or other binding agent. Target molecules include, but are not limited to, proteins, peptides, nucleic acids, carbohydrates, lipids, polysaccharides, glycoproteins, hormones, receptors, growth factors, and any portion or fragment of any of the foregoing, e.g., a short amino acid sequence or peptide (optionally glycosylated or otherwise modified) that is specifically bound by a binding agent (e.g., an antibody) or ligand of such target molecule.

The term "treating" or "treatment" covers the treatment of a disease or disorder (e.g., cancer), in a subject, such as a human, and includes: (i) inhibiting a disease or disorder, i.e., arresting its development; (ii) relieving a disease or disorder, i.e., causing regression of the disease or disorder; (iii) slowing progression of the disease or disorder; and/or (iv) inhibiting, relieving, or slowing progression of one or more symptoms of the disease or disorder. In some embodiments "treating" or "treatment" refers to the killing of cancer cells.

The term "kill" with respect to a cancer treatment is directed to include any type of manipulation that will lead to the death of that cancer cell or at least a portion of a population of cancer cells.

The term "dose" refers to an amount of an agent, e.g., the nanoparticle complexes or binding agents described herein, given to a patient in need thereof. The attending clinician will select an appropriate dose of the agent from the range based on, e.g., the patient's weight, age, health, stage of cancer, level of circulating VEGF in the case wherein the agent is an anti-VEGF antibody, and other relevant factors, all of which are well within the skill of the art.

The term "unit dose" refers to a dose of an agent, e.g., the nanoparticle complexes or binding agents described herein, that is given to the patient to provide a desired result. In some instances, the unit dose is sold in a sub-therapeutic formulation (e.g., 10% the therapeutic dose). The unit dose may be administered as a single dose or a series of sub-doses. The therapeutic dose for an agent for a given FDA-approved indication is recited in the prescribing information, for example the therapeutic dose of bevacizumab is 5 mg/kg to 15 mg/kg depending on the condition, and preferably a subtherapeutic dose ranges from 5% to 20% of the therapeutic dose. In an example such a subtherapeutic dose of an anti-VEGF antibody, e.g., bevacizumab, would range from 0.25 mg/kg to 3 mg/kg, more preferably from 0.5 to 2 mg/kg. The therapeutic dose for an antibody for a given indication where the antibody is not yet FDA approved or the antibody is not yet approved for that indication, will be the amount that correlates to the therapeutic dose that has been approved for other indications, and thus the subtherapeutic dose for the non-FDA approved indications is readily calculated as a percent of the therapeutic dose (e.g., 10% of the therapeutic dose). For example, the therapeutic dose and therefore the subtherapeutic dose of an antibody for the treatment of metastatic breast cancer correlates to the therapeutic dose for metastatic cancers in general that has been approved.

The term "triple-negative breast cancer" refers to a breast cancer characterized as estrogen receptor-negative, progesterone receptor-negative and human epidermal growth factor receptor-2-negative (HER2-negative). The determination of negative status of the estrogen, progesterone, and Her2/neu expression is readily determined by one of skill in the art, e.g., in accordance with the current accepted guidelines. For example, guidelines set forth by the American Society of Clinical Oncology (ASCO) and the College of American Pathologists (CAP) are widely accepted. The ASCO/CAP recommends testing by immunohistochemistry (IHC) or *in situ* hybridization (ISH) techniques. Further, a cancer is Her2 negative if a single test (or all tests) performed on a tumor specimen show: (a) IHC negative, IHC 1+ or IHC 0, or (b) ISH negative using single-probe ISH or dual-probe ISH. One of skill in the art would recognize that the triple negative cancer described herein does not include any cancer having an apparent histopathologic discordance as observed by the pathologist. Wolff, AC et al. J Clin Oncol. 2013 Nov 1:31(31):3997-4013. Cancer is ER-negative or PR-negative if <1 % of tumor cell nuclei are immunoreactive in the presence of evidence that the sample can express ER or PR (positive intrinsic controls are seen). Hammond, EH et al. J Oncol Pract. 2010 Jul; 6(4): 195-197.

Additionally, some terms used in this specification are more specifically defined below.

### Nanoparticle Complexes

As described herein, *in vitro* mixing of nanoparticles comprising a carrier protein and therapeutic agent, e.g., albumin, and paclitaxel (e.g., ABRAXANE^{®}) and a binding agent (e.g., an antibody, e.g., bevacizumab) can result in the formation of macromolecular complexes, the characteristics of which (e.g., size, antibody content, or therapeutic drug content) can be customized depending on need. In some cases, such macromolecular complexes can retain antibody mediated target binding specificity, can retain or exhibit enhanced chemotherapeutic tumor cell cytotoxicity, and can exhibit no additional toxicity beyond that of albumin/therapeutic nanoparticles alone. As also described herein, contacting said carrier protein/therapeutic agent, e.g., paclitaxel, nanoparticles with a binding agent as defined herein, e.g., an anti-VEGF antibody (e.g., bevacizumab) prior to administration to a human (e.g., a triple-negative breast cancer patient) can result in a complex that, when administered as a complex, has an increased ability to treat triple-negative breast cancer as compared to a treatment regimen that includes administering the nanoparticles and the binding agent, e.g., anti-VEGF antibody, separately in a manner that does not form the complexes, e.g., albumin/paclitaxel/anti-VEGF antibody complexes.

Methods for preparing nanoparticles of a carrier protein, e.g., albumin and chemotherapeutics, e.g., paclitaxel, can be found for example in U.S. Patent No. 8,853,260, U.S. Patent No. 7,923,536, U.S. Patent No. 7,820,788, and U.S. Patent No. 6,096,331. ABRAXANE^{®} is available from Celgene Corp. and is a nanoparticle formulation that combines paclitaxel with human albumin.

An anti-VEGF antibody such as bevacizumab is available from Genentech Inc., under the trade name AVASTIN^{®}. Bevacizumab is a recombinant humanized monoclonal IgG1 antibody which contains human framework regions (FRs) and the complementaritydetermining regions (CDRs) of a murine antibody that binds to vascular endothelial growth factor (VEGF). The anti-VEGF antibody fragment, ranibizumab, is available from Genentech Inc., under the trade name LUCENTIS^{®}.

The average diameter of the complexes can be from 0.1 µm to 1 µm. The average diameter of the complexes can be from 0.1 µm to 0.9 µm. The average diameter of the complexes can be from 0.1 µm to 0.5 µm. The average diameter of the complexes can be from 0.1 µm to 0.3 µm. The average diameter of the complexes can be from 0.15 µm to 0.3 µm. The average diameter of the complexes can be from 0.2 µm to 0.8 µm. The average diameter of the complexes can be from 0.2 µm to 0.5 µm. The average diameter of the complexes can be from 0.2 µm to 0.7 µm. The average diameter of the complexes can be from 0.3 µm to 0.5 µm.

In other embodiments, the nanoparticle complexes are larger, e.g., from greater than 800 nm to about 3.5 µm. In some embodiments, the particles are multimers of nanoparticles.

In some embodiments the nanoparticle complexes have average particle sizes of about 160 nm to about 225 nm either freshly made or after lyophilization and resuspension in an aqueous solution suitable for injection.

Preparations of carrier protein (e.g. albumin)/paclitaxel/antibody nanoparticle complexes provided herein, e.g., complexes having an average diameter that is between 1.1 µm and 0.9 µm, can be administered systemically (e.g., intravenously) for use in a method to treat a breast cancer located within a mammal's body.

In accordance with the invention, provided herein is a nanoparticle composition for use in a method for treating a patient suffering from triple-negative breast cancer which expresses or overexpresses a target molecule, wherein the method includes treating the patient with the composition comprising a therapeutic amount of nanoparticle complexes, the complexes comprising a) albumin; b) an effective amount of an antibody that binds to the antigen expressed by said cells of said breast cancer so as to provide directional guidance of the nanoparticle complexes to said cells; and c) an effective amount of paclitaxel, wherein the antigen expressed by said cells is cancer testis (CT) antigen, folate receptor alpha (FR-alpha), programmed-death-ligand 1 (PD-L1), programmed cell death protein 1 (PD-1), epidermal growth factor receptor (EGFR), C-kit, and/or basal cytokeratin, wherein the antibody is selected from an anti-cancer testis (CT) antigen antibody, an anti-folate receptor alpha (FR-alpha) antibody, an anti-programmed death-ligand 1 (PD-L1) antibody, an anti-programmed cell death protein 1 (PD-1) antibody, an anti-epidermal growth factor receptor (EGFR) antibody, an anti-C-kit antibody, and an anti-basal cytokeratin antibody, and wherein said antibodies are arranged on the outside surface of the nanoparticle complexes. A number of antigens have been found to be associated with TNBC in at least subsets of patients. Non-limiting examples of TNBC-associated antigens include cancer testis (CT) antigens, folate receptor alpha (FR-alpha), programmed death-ligand 1 (PD-L1), programmed cell death protein 1 (PD-1), epidermal growth factor receptor (EGFR), C-kit, and basal cytokeratins. CT antigens include those found in the CT Database (maintained by the Ludwig Institute for Cancer Research and accessible on the world wide web at cta.lncc.br/index.php). CT antigens of particular interest include, for example and without limitation, TSAG10, MAGEA family members (e.g., MAGEA2B, MAGEA3, MAGEA4, MAGEA5, MAGEA6, MAGEA9, MAGEA10, MAGEA12), NY-ESO-1/CTAG1B, CTAG2, PLAC1, and DKKL1. Basal cytokeratins include, for example and without limitation, CK-5, CK-14, and CK-17. Antibodies to each of these antigens are expressly contemplated by the present invention.

Anti-PD-L1 antibodies include, without limitation, BMS-936559 (made by Bristol-Myers Squibb), atezolizumab (TECENTRIQ^{®}, made by Genentech/Roche), durvalumab (IMFINZI^{®}, AstraZeneca UK Limited), and avelumab (BAVENCIO^{®}, EMD Serono, Inc.).

Anti-PD-1 antibodies include, without limitation, nivolumab (OPDIVO^{®}, Bristol-Myers Squibb) or pembrolizumab (KEYTRUDA^{®}, Merck).

Therapeutic agents, in addition to paclitaxel, that are useful in the methods described herein may be any therapeutic agent, e.g., a therapeutic agent selected from the group consisting of abiraterone, bendamustine, bortezomib, carboplatin, cabazitaxel, cisplatin, chlorambucil, dasatinib, docetaxel, doxorubicin, epirubicin, erlotinib, etoposide, everolimus, gefitinib, idarubicin, imatinib, hydroxyurea, imatinib, lapatinib, leuprorelin, melphalan, methotrexate, mitoxantrone, nedaplatin, nilotinib, oxaliplatin, pazopanib, pemetrexed, picoplatin, romidepsin, satraplatin, sorafenib, vemurafenib, sunitinib, teniposide, triplatin, vinblastine, vinorelbine, vincristine, and cyclophosphamide.

The carrier protein is albumin. In some embodiments the albumin is human serum albumin. In other embodiments the albumin is recombinant human serum albumin.

As an example not forming part of the claimed invention, provided herein is a method for increasing the duration of tumor uptake of a chemotherapeutic agent by administering the chemotherapeutic agent in a nanoparticle complex comprising a carrier protein and the chemotherapeutic agent and having surface complexation with an antibody, e.g., an antibody that specifically binds to an antigen on the tumor. The antigen may also be an antigen that is present on and shed by the tumor cell. In some embodiments, the subject receives a subtherapeutic amount of the antibody prior to or concurrently with such nanoparticle complexes.

Carrier protein/paclitaxel/anti-VEGF antibody complexes and methods of preparing the complexes are described, for example, in PCT Publication No. WO 2016/057554 A1, filed October 6, 2015. Example 1 below provides one example of a detailed protocol for making such complexes.

As an example, the antibodies are a substantially single layer of antibodies on all or part of the surface of the nanoparticle. Preferably, the antibodies associate with (complex with) the albumin via non-covalent bonds. Without being bound by theory, it is contemplated that binding agent interaction with the carrier protein, e.g., complexation of the binding agent to the carrier protein, occurs through an albumin-binding motif on the binding agents and/or an antibody-binding motif on the carrier protein. In some examples, the binding agent comprises an albumin-binding motif. In some examples, the carrier protein comprises an antibody-binding motif. Non-limiting examples of antibody-binding motifs can be found in PCT Publication No. WO2018027205, filed August 4, 2017.

### Anti-VEGF antibodies

In some examples, the binding agent is an anti-VEGF antibody, e.g., bevacizumab, or a biosimilar version thereof.

Bevacizumab (AVASTIN^{®}, Roche, USA) is a humanized monoclonal antibody that inhibits angiogenesis by blocking the action of vascular endothelial growth factor(VEGF). As such, bevacizumab can slow the growth of new blood vessels in tumors thereby inhibiting the tumors' ability to grow. Bevacizumab has been used to treat various cancers including, non-small cell lung cancer (NSCLC), metastatic colorectal cancer (mCRC), platinum-resistant ovarian cancer (prOC), advanced cervical cancer (CC), metastatic renal cell carcinoma (mRCC), and recurrent glioblastoma (rGBM). Several biosimilar versions of bevacizumab are currently being developed including ABP 215 (Amgen/Allergen, USA), BCD-021 (Biocad, Russia), BI 695502 (Boehringer Ingelheim, Germany), and PF-06439535 (Pfizer, USA), Cizumab (Hetero Drugs, India) among others.

In some examples, the sub-therapeutic amount of anti-VEGF antibody is selected from an amount consisting of about 1%, about 5%, about 10%, about 15%, about 20%, about 25%, about 30%, about 35%, about 40%, about 45%, about 50%, about 55% or about 60% of the therapeutic dosage of anti-VEGF antibody. In some examples the therapeutic amount of anti-VEGF antibody in the methods for the treatment of triple-negative breast cancer is 3 mg/kg to 30 mg/kg, 5 mg/kg to 20 mg/kg or 5 mg/kg to 15 mg/kg.

In some examples, the sub-therapeutic amount of anti-VEGF antibody is an amount which preferentially blocks circulating VEGF without blocking VEGF associated with tumor.

### Compositions for use in Treatment Methods

Any appropriate method can be used to administer an albumin-containing nanoparticle/antibody complexes provided herein (e.g., ABRAXANE^{®}/anti-VEGF antibody complexes) to a mammal in need thereof, e.g., a mammal having a triple-negative breast cancer that expresses or over expresses VEGF. For example, a composition containing albumin-containing nanoparticle/antibody complexes such as ABRAXANE^{®}/anti-VEGF polypeptide antibody complexes can be administered via injection (e.g., subcutaneous injection, intramuscular injection, intravenous injection, or intrathecal injection).

In another example, compositions for use in treatment of breast cancer can be used in methods wherein the mammal in need thereof has triple-negative breast cancer and the nanoparticle complexes administered to such mammal comprise an albumin-containing nanoparticle/antibody complex wherein the antibody binds a target molecule on the cancer cell that is not VEGF, e.g., the target molecule may be epidermal growth factor receptor (EGFR) and the anti-EGFR antibody may be Cetuximab/Erbitux; or the target molecule may be the transmembrane glycoprotein NMB (GPNMB), targeted by glembatumumab.

In some cases, the materials for use in the treatment of breast cancer provided herein can be used to treat triple-negative breast cancer in any type of mammal including, without limitation, mice, rats, dogs, cats, horses, cows, pigs, monkeys, and humans.

Before administering a composition containing an albumin-containing nanoparticle/antibody complex provided herein (e.g., ABRAXANE^{®}/anti-VEGF antibody complexes) to a mammal, the mammal can be assessed to determine whether or not the mammal has triple-negative breast cancer. Any appropriate method can be used to determine whether or not a mammal has triple-negative breast cancer. For example, a mammal (e.g., human) can be identified as having triple-negative breast cancer using standard diagnostic techniques. In some cases, a tissue biopsy can be collected and analyzed for the amount of estrogen receptor, epidermal growth factor receptor 2, and progesterone receptor expressed by cancer cells by immunohistochemistry or in situ hybridization to determine whether or not a mammal has triple-negative breast cancer.

After identifying a mammal as having triple-negative breast cancer, the mammal can be administered a composition containing carrier protein (e.g.. albumin)/paclitaxel/antibody complexes provided herein (e.g., ABRAXANE^{®}/anti-VEGF antibody complexes). For example, a composition containing ABRAXANE^{®}/anti-VEGF antibody complexes can be administered prior to or in lieu of surgical resection of a tumor. In some cases, a composition containing albumin chemotherapeutic-containing nanoparticle/antibody complexes provided herein (e.g., ABRAXANE^{®}/anti-VEGF antibody complexes) can be administered following resection of a tumor.

A composition containing albumin chemotherapeutic-containing nanoparticle/antibody complexes provided herein (e.g., ABRAXANE^{®}/anti-VEGF antibody complexes) can be administered to a mammal in any appropriate amount, at any appropriate frequency, and for any appropriate duration effective to achieve a desired outcome (e.g., to increase progression-free survival). In some cases, a composition containing albumin-containing nanoparticle/antibody complexes provided herein (e.g., ABRAXANE^{®}/anti- VEGF antibody complexes) can be administered to a mammal having triple-negative breast cancer to reduce the progression rate of the breast cancer by 5, 10, 25, 50, 75, 100, or more percent. For example, the progression rate can be reduced such that no additional cancer progression is detected and/or tumor size and/or volume is reduced following treatment. Any appropriate method can be used to determine whether or not the progression rate of breast cancer is reduced. For example, the progression rate of breast cancer can be assessed by imaging tissue at different time points and determining the amount of cancer cells present. The amounts of cancer cells determined within tissue at different times can be compared to determine the progression rate. After treatment as described herein, the progression rate can be determined again over another time interval. In some cases, the stage of cancer (e.g., triple-negative breast cancer) after treatment can be determined and compared to the stage before treatment to determine whether or not the progression rate was reduced. In some cases the overall survival of the subjects is improved.

As an example not forming part of the claimed invention, one aspect of this document features a method for treating a mammal having triple-negative breast cancer. The method comprises, or consists essentially of, administering to the mammal a composition comprising nanoparticle complexes containing albumin and paclitaxel complexed with an anti-VEGF antibody under conditions wherein the length of progression-free survival is increased. The mammal can be a human. The breast cancer can be triple-negative breast cancer. The composition can comprise anti-VEGF antibodies, e.g., bevacizumab, complexed with the nanoparticles. The composition can further comprise an alkylating agent, in some embodiments the alkylating agent is complexed with the nanoparticle complexes. The alkylating agent can be a platinum compound. The platinum compound can be carboplatin. The anti-VEGF antibody can be a humanized antibody. The anti-VEGF antibody can be a chimeric antibody. The anti-VEGF antibody may be bevacizumab or a biosimilar thereof. The composition can be administered by injection. The progression- free survival can be increased by 15 percent. The progression-free survival can be increased by 25 percent. The progression-free survival can be increased by 50 percent. The progression-free survival can be increased by 75 percent. The progression-free survival can be increased by 100 percent. The composition can be administered under conditions wherein the time to progression is increased.

In some cases, a composition containing albumin-containing nanoparticle/antibody complexes provided herein (e.g., ABRAXANE^{®}/anti-VEGF complexes) can be administered to a mammal having triple-negative breast cancer under conditions where progression-free survival is increased (e.g., by 5, 10, 25, 50, 75, 100, or more percent) as compared to the median progression-free survival of corresponding mammals having untreated triple-negative breast cancer or the median progression-free survival of corresponding mammals having triple-negative breast cancer treated with ABRAXANE^{®} and an antibody (e.g., an anti-VEGF antibody) without forming ABRAXANE^{®}/antibody complexes (e.g., without forming ABRAXANE^{®}/anti-VEGF antibody complexes). In some cases, a composition containing albumin-containing nanoparticle/antibody complexes provided herein (e.g., ABRAXANE^{®}/anti-VEGF antibody complexes) can be administered to a mammal having triple-negative breast cancer to increase progression-free survival by 5, 10, 25, 50, 75, 100, or more percent as compared to the median progression-free survival of corresponding mammals having triple-negative breast cancer and having received ABRAXANE^{®} or an antibody (e.g., an anti-VEGF antibody) alone. Progression-free survival can be measured over any length of time (e.g., one month, two months, three months, four months, five months, six months, or longer).

In some cases, a composition containing albumin-containing nanoparticle/antibody complexes provided herein (e.g., ABRAXANE^{®}/anti-VEGF antibody complexes) can be administered to a mammal having triple-negative breast cancer under conditions where the 8-week progression-free survival rate for a population of mammals is 65% or greater (e.g., 66%, 67%, 68%, 69%, 70%, 71%, 72%, 73%, 74%, 75%, 76%, 77%, 78%, 79%, 80% or greater) than that observed in a population of comparable mammals not receiving a composition containing albumin-containing nanoparticle/antibody complexes provided herein (e.g., ABRAXANE^{®}/anti-VEGF antibody complexes). In some cases, a composition containing albumin-containing nanoparticle/antibody complexes provided herein (e.g., ABRAXANE^{®}/anti-VEGF antibody complexes) can be administered to a mammal having triple-negative breast cancer under conditions where the median time to progression for a population of mammals is at least 150 days (e.g., at least 155, 160, 163, 165, or 170 days).

An effective amount of a composition containing albumin-containing nanoparticle/antibody complexes provided herein (e.g., ABRAXANE^{®}/anti-VEGF antibody complexes) can be any amount that reduces the progression rate of triple-negative breast cancer, increases the progression-free survival rate, or increases the median time to progression without producing significant toxicity to the mammal. Typically, an effective amount of ABRAXANE^{®} can be from about 50 mg/m2 to about 260 mg/m2 or about 75-175 mg/m2 or about 80 mg/m2, and an effective amount of an anti-VEGF antibody such as bevacizumab can be from about 3 mg/kg to 30 mg/kg, 5 mg/kg to about 20 mg/kg, or 5 mg/kg to 15mg/kg (e.g., about 10 mg/kg or 375 mg/m2). If a particular mammal fails to respond to a particular amount, then the amount of ABRAXANE^{®} or anti-VEGF antibody 20 can be increased by, for example, two fold. After receiving this higher concentration, the mammal can be monitored for both responsiveness to the treatment and toxicity symptoms, and adjustments made accordingly. The effective amount can remain constant or can be adjusted as a sliding scale or variable dose depending on the mammal's response to treatment. Various factors can influence the actual effective amount used for a particular application, which factors are routinely considered by those of skill in the art in determining the effective amount. For example, the frequency of administration, duration of treatment, use of multiple treatment agents, route of administration, and severity of the triple-negative breast cancer may require an increase or decrease in the actual effective amount administered. The complexes may comprise 100-1000 antibodies, e.g., about 400 to about 800 antibodies.

The frequency of administration can be any frequency that reduces the progression rate of triple-negative breast cancer, increases the progression-free survival rate, or increases the median time to progression without producing significant toxicity to the mammal. For example, the frequency of administration can be from about once a month to about three times a month, or from about twice a month to about six times a month, or from about once every two months to about three times every two months. The frequency of administration can remain constant or can be variable during the duration of treatment. A course of treatment with a composition containing ABRAXANE^{®}/anti-VEGF antibody complexes can include rest periods. For example, a composition containing ABRAXANE^{®}/anti-VEGF antibody complexes can be administered over a two week period followed by a two week rest period, and such a regimen can be repeated multiple times. As with the effective amount, those of skill in the art in determining the frequency of administration routinely consider various factors that can influence the actual frequency of administration used for a particular application. For example, the effective amount, duration of treatment, use of multiple treatment agents, route of administration, and severity of the triple-negative breast cancer may require an increase or decrease in administration frequency.

An effective duration for administering a composition provided herein can be any duration that reduces the progression rate of triple-negative breast cancer, increases the progression-free survival rate, or increases the median time to progression without producing significant toxicity to the mammal. Thus, the effective duration can vary from several days to several weeks, months, or years. In general, the effective duration for the treatment of triple-negative breast cancer can range in duration from several weeks to several months. In some cases, an effective duration can be for as long as an individual mammal is alive. Multiple factors can influence the actual effective duration used for a particular treatment. For example, an effective duration can vary with the frequency of administration, effective amount, use of multiple treatment agents, route of administration, and severity of the triple-negative breast cancer.

A composition containing albumin-chemotherapeutic agent-containing nanoparticle/antibody complexes provided herein (e.g., ABRAXANE^{®}/anti-VEGF antibody complexes) can be in any appropriate form. For example, a composition provided herein may be lyophilized or can be in the form of a solution or powder with or without a diluent to make an injectable suspension. A composition also can contain additional ingredients including, without limitation, pharmaceutically acceptable vehicles. A pharmaceutically acceptable vehicle can be, for example, saline, water, lactic acid, mannitol, or combinations thereof.

### Antibodies and Nanoparticle Complexes for use in Co-treatment Methods

As an example not forming part of the claimed invention, provided a method for treating a patient suffering from triple-negative breast cancer which expresses VEGF wherein said patient is co-treated with a sub-therapeutic amount of an anti-VEGF antibody and a composition comprising a nanoparticle complex comprising a) a carrier protein (e.g. albumin). b) an effective amount an anti-VEGF antibody) so as to provide directional guidance to the nanoparticle comples, and c) effective amount of paclitaxel, such that the administration of the sub-therapeutic amount of the anti-VEGF antibody enhances the efficacy of the nanoparticle complexes.

In some examples, the sub-therapeutic amount of anti-VEGF antibody is selected from an amount consisting of about 1%, about 5%, about 10%, about 15%, about 20%, about 25%, about 30%, about 35%, about 40%, about 45%, about 50%, about 55% or about 60% of the therapeutic dosage of anti-VEGF antibody. It is contemplated that administration of the sub-therapeutic amount of anti-VEGF antibody preferentially blocks circulating VEGF with minimal blocking of VEGF associated with a tumor. In some embodiments, the sub-therapeutic amount of anti-VEGF to be administered to the patient is determined by analyzing the level of circulating VEGF in the blood.

For the sake of clarification, "co-treatment" refers to treatment of the triple-negative breast cancer expressing VEGF (a soluble cytokine) with an anti-VEGF antibody prior to, concurrently with, or immediately after administration of the albumin/paclitaxel/anti-VEGF antibody nanoparticle complex, provided that the anti-VEGF antibody is capable of preferentially binding soluble VEGF.

In some examples the anti-VEGF antibody is administered prior to the albumin-bound chemotherapeutic/anti-VEGF antibody complex, for example, the anti-VEGF antibody can be administered minutes, hours or days prior to administration of the albumin/paclitaxel/anti-VEGF antibody nanoparticle complex. In some examples, the anti-VEGF antibody is administered between about 5 to about 59 minutes, about 10 to about 50 minutes, about 15 to about 45 minutes, about 20 to about 40 minutes, about 25 to about 35 minutes prior to administration of the albumin/paclitaxel/anti-VEGF antibody complex. In other examples, the anti-VEGF antibody can be administered about 1 hour, about 2hours, about 3 hours, about 4 hours, about 5 hours, about 6 hours, about 12 hours, about 24 hours, about 48 hours, about 72 hours, or longer prior to administration of the albumin/paclitaxel/anti-VEGF antibody complex. In other examples, the anti-VEGF antibody can be administered about 1 day, about 2 days, about 3 days, about 4 days, about 5 days, about 6 days, about 7 days, about 10 days, about 12 days, about 15 days, or longer prior to administration of the albumin/paclitaxel/anti-VEGF antibody complex.

In some examples, the anti-VEGF antibody can be administered concurrently with administration of the albumin/paclitaxel/anti-VEGF antibody complex, for example, within 10 minutes or less of each other.

In other examples, the anti-VEGF antibody can be administered subsequent to administration of the albumin/paclitaxel/anti-VEGF antibody complex, for example, within 2 hours after administration of the albumin/paclitaxel/anti-VEGF antibody complex, provided that the subsequent administration allows the antibody to preferentially bind the soluble VEGF.

In some examples, the anti-VEGF antibody is administered in a sub-therapeutic dose prior to administering the nanoparticle complex. In this example, the administration of the anti-VEGF antibody occurs about 0.5 to 48 hours prior to administration of the nanoparticle complexes.

In another example, the anti-VEGF antibody is administered between 0.5 hours prior to and up to 0.5 hours after administration of the nanoparticle complexes. In this example, it is contemplated that such administration will nevertheless result in binding of some of the circulating VEGF by the antibody.

As an example not forming part of the claimed invention, a method for treating a patient suffering from triple-negative breast cancer that expresses or overexpresses a target antigen (e.g., VEGF or a target antigen other than VEGF) that is in the blood stream, wherein said patient is treated with a sub-therapeutic amount of an antibody that binds to the target antigen and a composition comprising nanoparticle complexes, the complexes comprising a) a carrier protein; b) an antibody that binds to the target antigen, e.g., anti-VEGF antibodies; and c) paclitaxel, such that the administration of said sub-therapeutic amount of the antibody enhances the efficacy of said nanoparticle complexes. Further, without wishing to be bound by any theory, it is contemplated that administration of a subtherapeutic amount of the antibody alone prior to, concurrently with, or after administration of the nanoparticle complexes enhances the efficacy of the nanoparticle complexes. Without wishing to be bound by theory it is contemplated that the antibody alone binds to target antigens naturally expressed in the body and not bound to the tumor, e.g., soluble VEGF. Treatment with a sub-therapeutic amount of the antibody may allow for greater targeting of the nanoparticle complexes to the tumor, thereby decreasing the amounts of the albumin/paclitaxel/antibody complexes administered to a patient needed to achieve the effect achieved by the complexes without pre-treatment, or both.

As an example not forming part of the claimed invention, provided herein are methods for enhancing the efficacy of albumin/paclitaxel/anti-VEGF antibody nanoparticle complexes by administering the albumin/paclitaxel/anti-VEGF antibody nanoparticle complexes about 0.5 to 48 hours after pretreatment of a patient with a sub-therapeutic amount of anti-VEGF antibody.

Preferably, such nanoparticle complexes are administered about 24 hours after the subtherapeutic amount of anti-VEGF antibody.

In another example not forming part of the claimed invention, provided herein are methods for enhancing the therapeutic outcome in a patient suffering from triple-negative breast cancer expressing soluble VEGF. The patient selected is treated with nanoparticle complexes comprising albumin, paclitaxel and anti-VEGF antibodies, which method comprises treating said patient with a sub-therapeutic amount of said anti-VEGF antibody prior to any subsequent treatment with the nanoparticle complexes. In some embodiments, said antibodies of the nanoparticle complexes are integrated onto and/or into said nanoparticle complexes.

In another example not forming part of the claimed invention, provided herein are methods for enhancing the therapeutic outcome in a patient suffering from triple-negative breast cancer overexpressing soluble VEGF, said method comprising treating the patient with a sub-therapeutic amount of said anti-VEGF antibody and co-treating said patients with an effective amount of nanoparticle complexes comprising albumin-bound paclitaxel and anti-VEGF antibodies. In some examples, said antibodies of the nanoparticle complexes are integrated onto and/or into said nanoparticle complexes.

In yet another example, the antibody composition can be administered up to 2 hours post administration of the nanoparticle complexes.

In another example not forming part of the claimed invention, there are provided methods for enhancing the efficacy of albumin/paclitaxel/anti-VEGF antibody nanoparticle complexes by administering the albumin//paclitaxel/anti-VEGF antibody nanoparticle complexes about 0.5 to 48 hours after pretreatment of a patient with a subtherapeutic amount of anti-VEGF antibody. Preferably, such nanoparticle complexes are administered about 24 hours after administration of a sub-therapeutic amount of anti-VEGF antibody.

In another example not forming part of the claimed invention, there are provided methods for enhancing the therapeutic outcome in a patient suffering from triple-negative breast cancer expressing soluble VEGF which patient is selected to be treated with nanoparticle complexes comprising albumin-bound paclitaxel and anti-VEGF antibodies, which method comprises treating the patient with a sub-therapeutic amount of the anti-VEGF antibody prior to any subsequent treatment with the nanoparticle complexes.

In another example not forming part of the claimed invention, there are provided methods for enhancing the therapeutic outcome in a patient suffering from triple-negative breast cancer overexpressing soluble VEGF, said method comprising treating the patient with a sub-therapeutic amount of said anti-VEGF antibody and co-treating said patients with an effective amount of nanoparticle complexes comprising albumin-bound paclitaxel and anti-VEGF antibodies. In some examples, said antibodies of the nanoparticle complexes are integrated onto and/or into said complexes.

In another example not forming part of the claimed invention, there is provided a method for enhancing the therapeutic outcome in a patient suffering from triple-negative breast cancer expressing soluble VEGF, which patient is to be treated with nanoparticle complexes comprising albumin-bound paclitaxel and anti-VEGF antibodies, the method comprises treating said patient with a sub-therapeutic amount of said anti-VEGF antibody within +/- 0.5 hours of administration of said nanoparticle complexes.

In another example not forming part of the claimed invention, there is provided a method for enhancing the therapeutic outcome in a patient suffering from triple-negative breast cancer overexpressing soluble VEGF which patient has been treated with a sub-therapeutic amount of said anti-VEGF antibody, said method comprising treating said patients with an effective amount of nanoparticles complexes comprising albumin-bound paclitaxel and anti-VEGF antibodies within 0.5 hours of administration of said antibodies.

After administering a composition provided herein to a mammal, the mammal can be monitored to determine whether or not the composition provided beneficial results. For example, the mammal can be assessed after treatment to determine whether or not the progression rate of triple-negative breast cancer was reduced (e.g., stopped) or survival rate has increased. As described herein, any method can be used to assess progression and survival rates.

### Formulations - Antibodies

In one example not forming part of the claimed invention, the anti-VEGF is a unit-dose formulation of an anti-VEGF antibody which formulation comprises from about 1% to about 60% of a therapeutic dose of said antibody wherein said formulation is packaged so as to be administered as a unit dose. The unit-dose formulation of an anti-VEGF antibody comprises about 10% of a therapeutic dose of said antibody. For example 10% of a therapeutic dose of an anti-VEGF antibody, e.g., bevacizumab, may be 0.5 mg/kg to 5 mg/kg.

In some examples, the formulation comprises from about 5% to about 20% of a therapeutic dose of bevacizumab or a biosimilar version thereof. The therapeutic dose for bevacizumab for a given approved indication, e.g., treatment for metastatic colorectal cancer, nonsquamous non-small cell lung cancer, metastatic breast cancer, glioblastoma, and metastatic renal cell carcinoma, is recited in the prescribing information. In each case the therapeutic dose is from 5 to 15 mg/kg and preferably a subtherapeutic dose ranges from 5% to 20% of the therapeutic dose. In such a preferred embodiment such a subtherapeutic dose would range from 0.25 mg/kg to 3 mg/kg, more preferably from 0.5 to 2 mg/kg.

The unit-dose formulation of an anti-VEGF antibody can be about 1% to about 60%, about 5% to about 50%, about 10% to about 40%, about 15% to about 30%, about 20% to about 25%, of a therapeutic dose of the anti-VEGF antibody. Contemplated values include any value, sub-range, or range within any of the recited ranges, including endpoints.

In some examples, the anti-VEGF antibody is bevacizumab or a biosimilar version thereof, which formulation comprises from about 5% to about 20% of a therapeutic dose of bevacizumab or a biosimilar version thereof.

In another example not forming part of the claimed invention, provided herein is a formulation comprising an anti-VEGF antibody provided herein, and at least one pharmaceutically acceptable excipient.

In general, the unit-dose formulations provided herein can be formulated for administration to a patient by any of the accepted modes of administration. Various formulations and drug delivery systems are available in the art. See, e.g., Remington's Pharmaceutical Sciences, edited by E.W. Martin (Mack Publishing Company, 18th ed., 1995) and Remington: The Science and Practice of Pharmacy, edited by Allen, Loyd V., Jr, 22nd edition (2012).

In general, unit-dose formulations provided herein will be administered as pharmaceutical compositions by any one of the following routes: oral, systemic (e.g., transdermal, intranasal or by suppository), or parenteral (e.g., intramuscular, intravenous or subcutaneous) administration.

The unit-dose formulations may be comprised of, in general, an anti-VEGF antibody, optionally in combination with at least one pharmaceutically acceptable excipient. Acceptable excipients are non-toxic, aid administration, and do not adversely affect the therapeutic benefit of the claimed compounds. Such excipient may be any solid, liquid, semi-solid or, in the case of an aerosol composition, gaseous excipient that is generally available to one of skill in the art.

In other examples not forming part of the claimed invention, provided herein are unit-dose formulations of an anti-VEGF antibody, for example, bevacizumab or a biosimilar version thereof, which formulation comprises from about 1% to about 60% of a therapeutic dose of said antibody wherein said formulation is packaged so as to be administered as a unit dose.

### Formulations - Compositions Comprising Nanoparticle Complexes

In one aspect, the compositions comprising the nanoparticle complexes described herein are formulated for systemic delivery, e.g., intravenous administration.

In one aspect, the nanoparticle composition is formulated for direct injection into a tumor. Direct injection includes injection into or proximal to a tumor site, perfusion into a tumor, and the like. Because the nanoparticle composition is not administered systemically, a nanoparticle composition that is formulated for direct injection into a tumor may comprise any average particle size. Without being bound by theory, it is believed that larger particles (e.g., greater than 500 nm, greater than 1 µm, and the like) are more likely to be immobilized within the tumor, thereby providing what is believed to be a better beneficial effect.

In another aspect, provided herein is a composition comprising a compound provided herein, and at least one pharmaceutically acceptable excipient.

In general, the compounds provided herein can be formulated for administration to a patient by any of the accepted modes of administration. Various formulations and drug delivery systems are available in the art. See, e.g., Gennaro, A.R., ed. (2012) Remington's Pharmaceutical Sciences, 18th ed., Mack Publishing Co. (1995) and Remington: The Science and Practice of Pharmacy, edited by Allen, Loyd V., Jr, 22nd edition (2012).

In general, compounds provided herein will be administered as pharmaceutical compositions by any one of the following routes: oral, systemic (e.g., transdermal, intranasal or by suppository), or parenteral (e.g., intramuscular, intravenous or subcutaneous) administration.

The compositions are comprised of, in general, a compound of the present invention in combination with at least one pharmaceutically acceptable excipient. Acceptable excipients are non-toxic, aid administration, and do not adversely affect the therapeutic benefit of the claimed compounds. Such excipient may be any solid, liquid, semi-solid or, in the case of an aerosol composition, gaseous excipient that is generally available to one of skill in the art.

Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients may be selected from glycerol, propylene glycol, water, ethanol and various oils, including those of petroleum, animal, vegetable or synthetic origin, e.g., peanut oil, soybean oil, mineral oil, sesame oil, etc. Preferred liquid carriers, particularly for injectable solutions, include water, saline, aqueous dextrose, and glycols. Other suitable pharmaceutical excipients and their formulations are described in Remington's Pharmaceutical Sciences, edited by E.W. Martin (Mack Publishing Company, 18nd ed., 1995) and Remington: The Science and Practice of Pharmacy, edited by Allen, Loyd V., Jr, 22nd edition (2012).

The present formulations of antibody or composition comprising nanoparticle complexes may, if desired, be presented in a pack or dispenser device containing one or more unit dosage forms containing the active ingredient. Such a pack or device may, for example, comprise metal or plastic foil, such as a blister pack, or glass, and rubber stoppers such as in vials. The pack or dispenser device may be accompanied by instructions for administration. Compositions comprising antibodies or nanoparticle complexes as described herein formulated in a compatible pharmaceutical carrier may also be prepared, placed in an appropriate container, and labeled for treatment of an indicated condition.

### Kits

As an example not forming part of the claimed invention, provided herein are kits comprising: (a) an amount of an albumin/paclitaxel/anti-VEGF antibody complexes, (b) a unit dose of a sub-therapeutic amount of anti-VEGF antibody, and optionally (c) instructions for use.

In some examples, the kits can include lyophilized complexes of the albumin/paclitaxel/anti-VEGF antibody.

In some examples, the kit components can be configured in such a way that the components are accessed in their order of use. For example, in some aspects the kit can be configured such that upon opening or being accessed by a user, the first component available is the unit dose of a sub-therapeutic amount of anti-VEGF antibody, for example, in a first vial. A second container (e.g., a vial) comprising or containing an amount of the albumin/paclitaxel/anti-VEGF antibody complexes can then be accessed. As such, the kits can be intuitively configured in a way such that the first vial must be opened prior to the second vial being opened. It should be understood that in some examples, the order can be different, for example, where it is desired to administer the complex first, prior to the administration of the antibody. Also, it can be configured such that both are administered at the same time. Finally, it should be understood that additional vials or containers of either or both component(s) can be included, and configured for opening in any desired order. For example, the first vial could be antibody, the second vial could include complex, a third could include an antibody or complex, etc. It is contemplated that a kit configured in such a way would prevent, or at least help to prevent, the components from being administered in an order not intended by the instructions for use.

As an example not forming part of the claimed invention, provided herein is a kit of parts for administration of albumin-bound chemotherapeutic/anti-VEGF antibody complexes and a unit dose of a sub-therapeutic amount of anti-VEGF antibody; and optionally further comprising a dosing treatment schedule in a readable medium. In some examples, the dosing schedule includes the sub-therapeutic amount of anti-VEGF antibody required to achieve a desired average serum level is provided. In some examples, the kit of parts includes a dosing schedule that provides an attending clinician the ability to select a dosing regimen of the sub-therapeutic amount of anti-VEGF antibody based on the sex of the patient, mass of the patient, and the serum level that the clinician desires to achieve. In some examples, the dosing treatment is based on the level of circulating VEGF in the blood of the patient. In some examples, the dosing schedule further provides information corresponding to the volume of blood in a patient based upon weight (or mass) and sex of the patient. In an example, the storage medium can include an accompanying pamphlet or similar written information that accompanies the unit dose form in the kit. In an example, the storage medium can include electronic, optical, or other data storage, such as a nonvolatile memory, for example, to store a digitally-encoded machine-readable representation of such information.

The term "readable medium" as used herein refers to a representation of data that can be read, for example, by a human or by a machine. Non-limiting examples of human-readable formats include pamphlets, inserts, or other written forms. Non-limiting examples of machine-readable formats include any mechanism that provides (i.e., stores and/or transmits) information in a form readable by a machine (e.g., a computer, tablet, and/or smartphone). For example, a machine-readable medium includes read-only memory (ROM); random access memory (RAM); magnetic disk storage media; optical storage media; and flash memory devices. In one embodiment, the machine-readable medium is a CD-ROM. In one embodiment, the machine-readable medium is a USB drive. In one embodiment, the machine-readable medium is a Quick Response Code (QR Code) or other matrix barcode.

### EXAMPLES

The present disclosure is illustrated by administering to a subject in need thereof, e.g., a patient having a triple-negative breast cancer that also expresses VEGF, a composition comprising a therapeutically effective amount of nanoparticle complexes composed of albumin-bound paclitaxel (i.e., ABRAXANE^{®}) and bevacizumab (i.e., AVASTIN^{®}).

The present disclosure is also illustrated by pretreating a subject in need thereof, e.g., a patient having a triple-negative breast cancer that also expresses VEGF, with bevacizumab (i.e., AVASTIN^{®}) followed by administering to the subject a composition comprising a therapeutically effective amount of nanoparticle complexes composed of albumin-bound paclitaxel (i.e., ABRAXANE^{®}) and bevacizumab (i.e., AVASTIN^{®}).

One skilled in the art would understand that making and using the nanoparticle complexes, as well as administration of a co-treatment of bevacizumab, of the Examples are for the sole purpose of illustration, and that the present disclosure is not limited by this illustration.

Any abbreviation used herein, has normal scientific meaning. All temperatures are °C unless otherwise stated. Herein, the following terms have the following meanings unless otherwise defined:

| | | |
|---|---|---|
| ABX | = | ABRAXANE^{®}/(albumin-bound paclitaxel) |
| | | |
| ADC | = | antibody dependent chemotherapy |
| BEV | = | bevacizumab |
| BSA | = | bovine serum albumin |
| dH²O | = | distilled water |
| kg | = | kilogram |
| nm | = | nano molar |
| mg | = | milligram |
| ml or mL | = | milliliter |
| m² | = | square meters |
| mm³ | = | cubic millimeter |
| µg | = | microgram |
| µl | = | microliter |
| µm | = | micrometer/micron |
| PBS | = | Phosphate buffered saline |

### Example 1: Nanoparticle complex preparation

ABRAXANE^{®} (ABX) (10 mg) is suspended in bevacizumab (BEV) (4 mg [160 µl] unless otherwise indicated), and 840 µl of 0.9% saline is added to give a final concentration of 10 mg/ml and 2 mg/ml of ABX and BEV, respectively. The mixture is incubated for 30 minutes at room temperature (or at the temperature indicated) to allow particle formation. For Mastersizer experiments to measure particle size of ABX:BEV complexes, 10 mg of ABX is suspended in BEV at concentrations of 0 to 25 mg/ml. Complexes of ABX with rituximab (0-10 mg/ml) or trastuzumab (0-22 mg/ml) were formed under similar conditions.

For use in humans, the ABX:BEV complexes may be prepared by obtaining the dose appropriate number of 4 mL vials of 25 mg/mL BEV and diluting each vial per the following directions to 4 mg/mL. The dose appropriate number of 100 mg vials of ABX can be prepared by reconstituting to a final concentration containing 10 mg/mL ABX nanoparticles. Using a sterile 3 mL syringe, 1.6 mL (40 mg) of bevacizumab (25 mg/mL) can be withdrawn and slowly injected, over a minimum of 1 minute, onto the inside wall of each of the vials containing 100 mg of ABX. The bevacizumab solution should not be injected directly onto the lyophilized cake as this will result in foaming. Then, using a sterile 12 mL sterile syringe, 8.4 mL 0.9% Sodium Chloride Injection, USP, can be withdrawn and slowly injected, over a minimum of 1 minute, 8.4 mL onto the inside wall of each vial containing ABX 100 mg and BEV 40 mg. Once the addition of BEV 1.6 mL and 0.9% Sodium Chloride Injection, USP 8.4 mL is completed, each vial can be gently swirled and/or inverted slowly for atleast 2 minutes until complete dissolution of any cake/powder occurs. Generation of foam should be avoided. At this point, the concentration of each vial should be 100 mg/10 mL ABX and 40 mg/10 mL BEV. The vials containing the ABX and BEV should sit for 60 minutes. The vial(s) should be gently swirled and/or inverted every 10 minutes to continue to mix the complex. After 60 minutes has elapsed, the calculated dosing volume of ABX and BEV should be withdrawn from each vial and slowly added to an empty viaflex bag. An equal volume of 0.9% Sodium Chloride Injection, USP is then added to make the final concentration of ABX 5 mg/mL and BEV 2 mg/mL. The bag should then be gently swirled and/or inverted slowly for 1 minute to mix. The ABX:BEV nanoparticles can be stored for up to 4 hours at room temperature following final dilution.

### Example 2: Treatment of triple-negative breast cancer with ABX/BEV complexes reduces tumor size

Athymic nude mice are injected with 2 to 4 x 10⁶ triple-negative breast cancer cells in the right flank and PBS, 12 mg/kg BEV, 30 mg/kg ABX, or AB160, is administered to the mice, or 1.2 mg/kg BEV is administered to the mice and, 24 hr. later, AB160 is administered to the mice. AB160 is prepared as described in WO 2016/057554 A1 and Example 1 above. The tumor size is tracked over 40 days. Data is represented as tumor volume in mm3. Only mice treated with AB160 (with or without pretreatment with BEV) show a reduction in average tumor volume.

It is expected that pretreatment with a subtherapeutic dose of anti-VEGF antibodies (BEV) improves the efficacy of the AB160 nanoparticle complexes. Without wishing to be bound by theory, pretreatment with a subtherapeutic amount of BEV may clear systemic levels of VEGF, leaving a greater relative concentration of VEGF at the tumor such that tumorassociated VEGF targeting by the AB160 nanoparticles is more effective.

Tumors are measured on day 15 following treatment with either saline (PBS), AVASTIN^{®} (BEV), ABRAXANE^{®} (ABX), AB160, or a pretreatment of BEV one day before AB160 (BEV+AB160). A 10% sub-therapeutic dose of BEV, as compared to the dose give to the BEV alone or AB 160 cohort, is given to the BEV+AB 160 cohort 24 hours prior to administration of the AB 160. Pretreatment with BEV+AB 160, will increase survival. Both treatment groups will have substantially greater survival than the PBS, BEV-only or ABX-only treatment groups. Survival is again assessed at day 40.

### Example 3: Fluorescence over time of AlexaFluor 750 labeled nanoparticles

Mice receive IV injections of equal amounts of either labeled ABRAXANE^{®}, or nanoparticles of ABRAXANE^{®} having surface complexation with bevacizumab (BEV) as per Example 1 above (AB 160); one AB 160 group of mice receive a pretreatment 1.2 mg/kg of bevacizumab. Fluorescent imagery is done at an excitation/emission spectrum of 710/760. Regions of interest (ROI) in the mice are assigned by software to track tumor accumulation based on a fluorescence threshold. Fluorescence per unit area of background ROIs and tumor ROIs for all three treatment groups are determined at 24, 29, and 48 hours post injection.

Pretreatment with BEV will result in higher levels of tumor fluorescence as compared to AB160 alone or ABRAXANE alone. Pretreatment with BEV and use of ABRAXANE^{®} nanoparticles having surface complexation with BEV provides for a method for increasing the duration of tumor uptake of albumin containing a chemotherapeutic agent both at 24 hours and 48 hours. Without being limited to any theory, the antibody coating of the albumin nanoparticles imparts stability possibly by reducing liver or kidney clearance and/or by reducing protease degradation of the albumin carrier. This approach allows targeting antibodies to complex with a protein carrier such as albumin thereby providing prolonged targeting of such antibody-bound complexes to the tumor.

### Example 4: Nanoparticles having a size of 225 nm

To make a nanoparticle having a size of 225 nm, the particles are prepared in accordance with Example 1 but the ratio of BEV to ABRAXANE^{®} was 4:5, i.e., 4 parts BEV and 5 parts ABRAXANE. This ratio produced nanoparticles having a size of 225 nm (AB225). The effect of AB225 is assayed in animals as set forth above. The survival of the mice treated with a single dose of saline, BEV24 (24 mg/kg), ABX30 (30 mg/kg), AB160 (12 mg/kg BEV and 30 mg/kg ABX) and AB225 (24 mg/kg BEV and 30 mg/kg ABX) and with AB 160 with a BEV (1.2 mg/kg) pretreatment is determined at 30 days.

## Claims

1. A nanoparticle composition for use in the treatment of a breast cancer in a patient, wherein the cells of the breast cancer are characterized as having a triple negative phenotype and further characterized as expressing an antigen, the treatment comprising administering to the patient said composition, wherein said composition comprises a therapeutic amount of nanoparticle complexes, the complexes comprising:
a) albumin;
b) an effective amount of an antibody that binds to the antigen expressed by said cells of said breast cancer so as to provide directional guidance of the nanoparticle complexes to said cells; and
c) an effective amount of paclitaxel,
wherein the antigen expressed by said cells is cancer testis (CT) antigen, folate receptor alpha (FR-alpha), programmed-death-ligand 1 (PD-L1), programmed cell death protein 1 (PD-1), epidermal growth factor receptor (EGFR), C-kit, and/or basal cytokeratin, wherein the antibody is selected from an anti-cancer testis (CT) antigen antibody, an anti-folate receptor alpha (FR-alpha) antibody, an anti-programmed death-ligand 1 (PD-L1) antibody, an anti-programmed cell death protein 1 (PD-1) antibody, an anti-epidermal growth factor receptor (EGFR) antibody, an anti-C-kit antibody, and an anti-basal cytokeratin antibody, and wherein said antibodies are arranged on the outside surface of the nanoparticle complexes.

2. The nanoparticle composition for use of claim 1, wherein said antigen expressed by said cells is said CT antigen, and wherein the CT antigen is selected from TSAG10, a MAGE family member, NY-ESO-1/CTAG1B, CTAG2, PLAC1, and DKKL1.

3. The nanoparticle composition for use of claim 2, wherein said CT antigen is said MAGE family member, and wherein the MAGE family member is selected from MAGEA2B, MAGEA3, MAGEA4, MAGEA5, MAGEA6, MAGEA9, MAGEA10, and MAGEA12.

4. The nanoparticle composition for use of claim 1, wherein said antigen expressed by said cells is said basal cytokeratin, and wherein the basal cytokeratin is CK-5, CK-14, or CK-17.

5. The nanoparticle composition for use of claim 1, wherein said antibody is said anti-PD-L1 antibody, and wherein the anti-PD-L1 antibody is atezolizumab, durvalumab, or avelumab.

6. The nanoparticle composition for use of claim 1, wherein said antibody is said anti-PD-1 antibody, and wherein the anti-PD-1 antibody is nivolumab or pembrolizumab.

7. The nanoparticle composition for use of claim 1, wherein said antibody is said anti-EGFR antibody, and wherein the anti-EGFR antibody is cetuximab or panitumumab.

8. The nanoparticle composition for use of any one of claims 1-7, wherein the albumin is human serum albumin, optionally wherein the albumin is recombinant human serum albumin.

9. The nanoparticle composition for use of any one of claims 1-8, wherein each of said nanoparticle complexes comprises between 100 and 1,000 antibodies.

10. The nanoparticle composition for use of any one of claims 1-9, wherein the antibodies are arranged into a substantially single layer of antibodies on all or part of a surface of the nanoparticle complexes.

11. The nanoparticle composition for use of any one of claims 1-10, wherein the average size of nanoparticle complexes in the nanoparticle composition is from greater than 800 nm to 3.5 µm, or wherein the average size of the nanoparticle complexes is 100 nm to less than 1 micron, optionally wherein the average size of the nanoparticle complexes is between 130 nm and 800 nm, optionally wherein said nanoparticle complexes have an average size of approximately 160 nm.

12. The nanoparticle composition for use of any one of claims 1-11, wherein the cells of the breast cancer have not previously been treated with an anti-VEGF antibody.

13. The nanoparticle composition for use of any one of claims 1-12, wherein the cell of the breast cancer is resistant to conventional hormonal therapies and/or trastuzumab therapy.

14. The nanoparticle composition for use of any one of claims 1-13, wherein the composition is administered in a dose that provides 75-175 mg/m² of the paclitaxel to the patient.

15. The nanoparticle composition for use of any one of claims 1-14, wherein the patient is pretreated with a subtherapeutic dose of the antibody prior to administration of the nanoparticle composition.

## Patentansprüche

1. Nanopartikelzusammensetzung zur Verwendung bei der Behandlung eines Brustkrebses bei einer Patientin, wobei die Zellen des Brustkrebses **dadurch gekennzeichnet sind, dass** sie einen dreifach negativen Phänotyp aufweisen und ferner **dadurch gekennzeichnet sind, dass** sie ein Antigen exprimieren, wobei die Behandlung Verabreichen der Zusammensetzung an die Patientin umfasst, wobei die Zusammensetzung eine therapeutische Menge an Nanopartikelkomplexen umfasst, wobei die Komplexe Folgendes umfassen:
a) Albumin;
b) eine wirksame Menge eines Antikörpers, der an das Antigen bindet, das von den Zellen des Brustkrebses exprimiert wird, um so eine gerichtete Führung der Nanopartikelkomplexe zu den Zellen bereitzustellen; und
c) eine wirksame Menge Paclitaxel,
wobei das von den Zellen exprimierte Antigen Krebs-Hoden-(CT)-Antigen, Folatrezeptor-alpha (FR-alpha), programmierter Tod-Ligand 1 (PD-L1), programmiertes Zelltod-Protein 1 (PD-1), epidermaler Wachstumsfaktorrezeptor (EGFR), C-Kit und/oder basales Zytokeratin ist, wobei der Antikörper ausgewählt ist aus einem Antikörper gegen Krebs-Hoden-(CT)-Antigen, einem Antikörper gegen Folatrezeptor-alpha (FR-alpha), einem Antikörper gegen programmierten Tod-Ligand 1 (PD-L1), einem Antikörper gegen programmiertes Zelltod-Protein 1 (PD-1), einem Antikörper gegen epidermalen Wachstumsfaktorrezeptor (EGFR), einem Antikörper gegen C-Kit und einem Antikörper gegen basales Zytokeratin und wobei die Antikörper auf der äußeren Oberfläche der Nanopartikelkomplexe angeordnet sind.

2. Nanopartikelzusammensetzung zur Verwendung nach Anspruch 1, wobei das von den Zellen exprimierte Antigen das CT-Antigen ist und wobei das CT-Antigen ausgewählt ist aus TSAG10, einem Mitglied der MAGE-Familie, NY-ESO-1/CTAG1B, CTAG2, PLAC1 und DKKL1.

3. Nanopartikelzusammensetzung zur Verwendung nach Anspruch 2, wobei das CT-Antigen das Mitglied der MAGE-Familie ist und wobei das Mitglied der MAGE-Familie ausgewählt ist aus MAGEA2B, MAGEA3, MAGEA4, MAGEA5, MAGEA6, MAGEA9, MAGEA10 und MAGEA12.

4. Nanopartikelzusammensetzung zur Verwendung nach Anspruch 1, wobei das von den Zellen exprimierte Antigen das basale Zytokeratin ist und wobei das basale Zytokeratin CK-5, CK-14 oder CK-17 ist.

5. Nanopartikelzusammensetzung zur Verwendung nach Anspruch 1, wobei der Antikörper der Antikörper gegen PD-L1 ist und wobei der Antikörper gegen PD-L1 Atezolizumab, Durvalumab oder Avelumab ist.

6. Nanopartikelzusammensetzung zur Verwendung nach Anspruch 1, wobei der Antikörper der Antikörper gegen PD-1 und wobei der Antikörper gegen PD-1 Nivolumab oder Pembrolizumab ist.

7. Nanopartikelzusammensetzung zur Verwendung nach Anspruch 1, wobei der Antikörper der Antikörper gegen EGFR ist und wobei der Antikörper gegen EGFR Cetuximab oder Panitumumab ist.

8. Nanopartikelzusammensetzung zur Verwendung nach einem der Ansprüche 1 bis 7, wobei das Albumin humanes Serumalbumin ist, wobei das Albumin gegebenenfalls rekombinantes humanes Serumalbumin ist.

9. Nanopartikelzusammensetzung zur Verwendung nach einem der Ansprüche 1 bis 8, wobei jeder der Nanopartikelkomplexe zwischen 100 und 1.000 Antikörper umfasst.

10. Nanopartikelzusammensetzung zur Verwendung nach einem der Ansprüche 1 bis 9, wobei die Antikörper in einer im Wesentlichen einzelnen Schicht von Antikörpern auf einer gesamten Oberfläche der Nanopartikelkomplexe oder einem Teil davon angeordnet sind.

11. Nanopartikelzusammensetzung zur Verwendung nach einem der Ansprüche 1 bis 10, wobei die durchschnittliche Größe von Nanopartikelkomplexen in der Nanopartikelzusammensetzung von mehr als 800 nm bis 3,5 µm beträgt oder wobei die durchschnittliche Größe der Nanopartikelkomplexe 100 nm bis weniger als 1 Mikron beträgt, wobei gegebenenfalls die durchschnittliche Größe der Nanopartikelkomplexe zwischen 130 nm und 800 nm beträgt, wobei gegebenenfalls die Nanopartikelkomplexe eine durchschnittliche Größe von ungefähr 160 nm aufweisen.

12. Nanopartikelzusammensetzung zur Verwendung nach einem der Ansprüche 1 bis 11, wobei die Zellen des Brustkrebses zuvor nicht mit einem Antikörper gegen VEGF behandelt wurden.

13. Nanopartikelzusammensetzung zur Verwendung nach einem der Ansprüche 1 bis 12, wobei die Zelle des Brustkrebses gegenüber herkömmlichen Hormontherapien und/oder einer Trastuzumab-Therapie resistent ist.

14. Nanopartikelzusammensetzung zur Verwendung nach einem der Ansprüche 1 bis 13, wobei die Zusammensetzung in einer Dosis verabreicht wird, die der Patientin 75 bis 175 mg/m² Paclitaxel bereitstellt.

15. Nanopartikelzusammensetzung zur Verwendung nach einem der Ansprüche 1 bis 14, wobei die Patientin vor Verabreichung der Nanopartikelzusammensetzung mit einer subtherapeutischen Dosis des Antikörpers vorbehandelt wird.

## Revendications

1. Composition de nanoparticules destinée à être utilisée dans le traitement d'un cancer du sein chez un patient, dans laquelle les cellules du cancer du sein sont caractérisées comme ayant un phénotype triple négatif et caractérisées en outre comme exprimant un antigène, le traitement comprenant l'administration au patient de ladite composition, ladite composition comprenant une quantité thérapeutique de complexes de nanoparticules, les complexes comprenant :
a) de l'albumine ;
b) une quantité efficace d'un anticorps qui se lie à l'antigène exprimé par lesdites cellules dudit cancer du sein de manière à fournir un guidage directionnel des complexes de nanoparticules vers lesdites cellules ; et
c) une quantité efficace de paclitaxel,
dans laquelle l'antigène exprimé par lesdites cellules est un antigène testiculaire cancéreux (CT), le récepteur alpha du folate (FR-alpha), le ligand 1 de mort programmée (PD-L1), la protéine 1 de mort cellulaire programmée (PD-1), le récepteur du facteur de croissance épidermique (EGFR), C-kit et/ou la cytokératine basale, l'anticorps étant sélectionné parmi un anticorps antiantigène testiculaire cancéreux (CT), un anticorps antirécepteur alpha du folate (FR-alpha), un anticorps antiligand 1 de mort programmée (PD-L1), un anticorps antiprotéine 1 de mort cellulaire programmée (PD-1), un anticorps anti-récepteur du facteur de croissance épidermique (EGFR), un anticorps anti-C-kit et un anticorps anti-cytokératine basale, et dans laquelle lesdits anticorps sont disposés sur la surface extérieure des complexes de nanoparticules.

2. Composition de nanoparticules destinée à être utilisée selon la revendication 1, dans laquelle ledit antigène exprimé par lesdites cellules est ledit antigène CT, et dans laquelle l'antigène CT est choisi parmi TSAG10, un membre de la famille MAGE, NY-ESO-1/CTAG1B, CTAG2, PLAC1 et DKKL1.

3. Composition de nanoparticules destinée à être utilisée selon la revendication 2, dans laquelle ledit antigène CT est ledit membre de la famille MAGE, et dans laquelle le membre de la famille MAGE est choisi parmi MAGEA2B, MAGEA3, MAGEA4, MAGEA5, MAGEA6, MAGEA9, MAGEA10, et MAGEA12.

4. Composition de nanoparticules destinée à être utilisée selon la revendication 1, dans laquelle ledit antigène exprimé par lesdites cellules est ladite cytokératine basale, et dans laquelle la cytokératine basale est CK-5, CK-14 ou CK-17.

5. Composition de nanoparticules destinée à être utilisée selon la revendication 1, dans laquelle ledit anticorps est ledit anticorps anti-PD-L1, et dans laquelle l'anticorps anti-PD-L1 est atézolizumab, durvalumab ou avélumab.

6. Composition de nanoparticules destinée à être utilisée selon la revendication 1, dans laquelle ledit anticorps est ledit anticorps anti-PD-1, et dans laquelle l'anticorps anti-PD-1 est nivolumab ou pembrolizumab.

7. Composition de nanoparticules destinée à être utilisée selon la revendication 1, dans laquelle ledit anticorps est ledit anticorps anti-EGFR, et dans laquelle l'anticorps anti-EGFR est le cétuximab ou le panitumumab.

8. Composition de nanoparticules destinée à être utilisée selon l'une quelconque des revendications 1 à 7, dans laquelle l'albumine est la sérum-albumine humaine, facultativement dans laquelle l'albumine est la sérum-albumine humaine recombinante.

9. Composition de nanoparticules destinée à être utilisée selon l'une quelconque des revendications 1 à 8, dans laquelle chacun desdits complexes de nanoparticules comprend entre 100 et 1 000 anticorps.

10. Composition de nanoparticules destinée à être utilisée selon l'une quelconque des revendications 1 à 9, dans laquelle les anticorps sont disposés en une couche sensiblement unique d'anticorps sur la totalité ou une partie d'une surface des complexes de nanoparticules.

11. Composition de nanoparticules pour utilisation selon l'une quelconque des revendications 1 à 10, dans laquelle la taille moyenne des complexes de nanoparticules dans la composition de nanoparticules est de plus de 800 nm à 3,5 µm, ou dans laquelle la taille moyenne des complexes de nanoparticules est de 100 nm à moins de 1 micron, facultativement dans laquelle la taille moyenne des complexes de nanoparticules est comprise entre 130 nm et 800 nm, facultativement dans laquelle lesdits complexes de nanoparticules ont une taille moyenne d'environ 160 nm.

12. Composition de nanoparticules destinée à être utilisée selon l'une quelconque des revendications 1 à 11, dans laquelle les cellules du cancer du sein n'ont pas été préalablement traitées par un anticorps anti-VEGF.

13. Composition de nanoparticules destinée à être utilisée selon l'une quelconque des revendications 1 à 12, dans laquelle la cellule du cancer du sein est résistante aux thérapies hormonales classiques et/ou à une thérapie par trastuzumab.

14. Composition de nanoparticules destinée à être utilisée selon l'une quelconque des revendications 1 à 13, dans laquelle la composition est administrée en une dose qui fournit 75-175 mg/m² du paclitaxel au patient.

15. Composition de nanoparticules destinée à être utilisée selon l'une quelconque des revendications 1 à 14, dans laquelle le patient est prétraité par une dose subthérapeutique de l'anticorps avant l'administration de la composition de nanoparticules.
